(19) Europäisches Patentamt

European Patent Office

Office européen des brevets

(11) Veröffentlichungsnummer : **0 365 567 B1**

(12) **EUROPÄISCHE PATENTSCHRIFT**

(45) Veröffentlichungstag der Patentschrift :
**22.01.92 Patentblatt 92/04**

(21) Anmeldenummer : **88905686.7**

(22) Anmeldetag : **07.07.88**

(86) Internationale Anmeldenummer :
**PCT/DE88/00418**

(87) Internationale Veröffentlichungsnummer :
**WO 89/00686 26.01.89 Gazette 89/03**

(51) Int. Cl.⁵ : **G01N 27/12**

(54) **HALBLEITER FÜR EINEN RESISTIVEN GASSENSOR MIT HOHER ANSPRECHGESCHWINDIGKEIT.**

(30) Priorität : **11.07.87 DE 3723051**

(43) Veröffentlichungstag der Anmeldung :
**02.05.90 Patentblatt 90/18**

(45) Bekanntmachung des Hinweises auf die
Patenterteilung :
**22.01.92 Patentblatt 92/04**

(84) Benannte Vertragsstaaten :
**AT BE CH DE FR GB IT LI NL SE**

(56) Entgegenhaltungen :
**EP-A- 0 055 104**
**GB-A- 2 149 121**
**US-A- 3 951 603**
**US-A- 4 677 414**

(73) Patentinhaber : **ROTH-Technik GmbH & Co.**
**Forschung für Automobil- und Umwelttechnik**
**Max-Roth-Strasse**
**W-7560 Gaggenau (DE)**

(72) Erfinder : **HÄFELE, Edelbert**
**Talwiesenstr. 13**
**W-7500 Karlsruhe 1 (DE)**
Erfinder : **HÄRDTL, Karl-Heinz**
**Prof.-Eichmannstr. 27**
**W-6729 Hagenbach (DE)**
Erfinder : **MÜLLER, Andreas**
**Rottmannstr. 30**
**W-6900 Heidelberg (DE)**
Erfinder : **SCHÖNAUER, Ulrich**
**Jollystr. 1**
**W-7500 Karlsruhe (DE)**

(74) Vertreter : **Säger, Manfred, Dipl.-Ing.**
**Säger & Partner Postfach 810 809**
**W-8000 München 80 (DE)**

## Beschreibung

Die Erfindung betrifft einen Halbleiter für einen resistiven Gassensor mit hoher Ansprechgeschwindigkeit entsprechend dem Oberbegriff von Anspruch 1 und ein Verfahren zu dessen Herstellung.

Zur Messung des Partialdrucks von Sauerstoff und reduzierenden Gasen (wie z.B. NO, CO, $C_3H_8$ etc.) eignen sich als Halbleiter spezielle Mischoxide, die sogenannten Perowskite, in besonderer Weise.

Gattungsgemäße Gassensor-Elemente sind aus der EP-A-0055104 bekannt. Hier wird ein poröses Sensorelement mit verbesserter Ansprechgeschwindigkeit vorgeschlagen, das durch die allgemeine Zusammensetzung $A'_xA_{1-x-z1}B'_yB_{1-y-z2}O_3$ gekennzeichnet ist, wobei A für La, Ce, Pr und Nd, A' für Ca, Sr, Ba, B für Co und B' für Fe, Mn, V und Ti steht. Die Werte von x sollen im Bereich von 0,6 bis 0,95 und von y im Bereich von 0,2 bis 0,9 liegen, wobei gelten muß : x = 0,5 (1 + y). In einem Beispiel wird eine Schichtdicke von 2,5 mm angegeben.

Diese Sensorelemente zeigen jedoch noch kein völlig befriedigendes Ansprechverhalten. Wesentliche Gründe hierfür sind die starke Dotierung, bei der die Konzentration der dotierten Elemente größenordnungsgemäß im Bereich der Stammelemente liegt, und die große Schichtdicke, deren nachteilige Auswirkungen auch dadurch nicht voll kompensiert werden können, daß das Sensorelement porös ausgebildet wird.

Aus der GB-A-2149121 sind ferner Gassensoren derselben allgemeinen Zusammensetzung bekannt, bei denen anstelle von Co Sn vorgeschlagen wird. Für die Indices x und y werden Werte im Bereich von 0 bis 1 angegeben. Als mögliches Herstellungsverfahren für solche Gassensoren wird zwar die Siebdrucktechnik erwähnt, jedoch wird auch hier in einem Beispiel eine Schichtdicke von 2 mm genannt. Der Gassensor soll porös sein, um das Oberfläche/Volumenverhältnis zu vergrößern.

Grundsätzlich sind poröse Gassensoren weniger geeignet, da sich in den Poren leicht Verschmutzungen ablagern können, die sich nur schwer entfernen lassen und die Eigenschaften des Sensors stark beeinflussen können.

Perowskit-Halbleiter für Gassensoren und deren Herstellung durch Sinterung wurden weiterhin von Obayashi et al in US-PS 3,951,603 und US-PS 3,953,173, von Sakurai et al in US-PS 4,044,601 und von Perry et al in US-PS 4,221,827 beschrieben.

Im Gegensatz zu Halbleitern aus Zinnoxid o.ä. Materialien, bei denen sich in Abhängigkeit vom Meßgas-Partialdruck der Oberflächenwiderstand ändert, beruht die Widerstandsänderung in Halbleitern aus Perowskiten auf einem Volumeneffekt ; d.h., der Halbleiter-Widerstand ändert sich in Abhängigkeit von der Meßgaskonzentration durch Diffusion von Sauerstoff im Halbleiter.

Die Zugabe von reduzierenden Gasen im Meßgas bewirkt eine Verringerung der Sauerstoffkonzentration im Halbleiter durch die Diffusion von Sauerstoff an die Festkörperoberfläche ; die Zugabe von Sauerstoff im Meßgas bewirkt eine entsprechende Erhöhung der Sauerstoffkonzentration im Halbleiter durch Diffusion von Sauerstoff in den Festkörper.

Weil jedoch nach Änderung des Meßgaspartialdrucks eine Sauerstoffdiffusion in oder aus dem Halbleitermaterial erfolgen muß, sollen solche Halbleiter im Interesse einer hohen Ansprechgeschwindigkeit möglichst dünn sein, denn die Diffusionszeit des Sauerstoffs ist dem Quadrat der Schichtdicke proportional. Solche Halbleiter sind gegen oberflächliche Verschmutzung weitgehend unempfindlich und können auch in rauherer Umgebung eingesetzt werden.

Durch Sinterung lassen sich Halbleiter mit minimalen Schichtdicken zwischen 20 und 50 $\mu$m, meist jedoch 500 $\mu$m, herstellen. Das Sinterverfahren wird häufig als einfaches Herstellungsverfahren für Gassensor-Halbleiter in kleiner Stückzahl angewendet.

Bei Serienfertigung fallen die hohen Energiekosten durch die lange Sinterdauer und der zusätzliche Aufwand für die erforderliche Nachbehandlung durch Sägen, Schleifen und Polieren ins Gewicht.

Für höhere Anforderungen an die Ansprechgeschwindigkeit könnten die Halbleiter in Dünnfilmtechnik hergestellt werden. Auf diese Weise wurden bisher Dielektrika auf Kondensatorplatten aufgebracht. Dabei wurden die den Halbleiter bildenden Substanzen durch Aufstäuben (Sputtern) auf eine Unterlage aufgebracht. Damit ließen sich sehr kleine Schichtdicken realisieren.

Das Verfahren ist jedoch — bedingt durch die Hochvakuumtechnik — sehr aufwendig und damit teuer, so daß es für eine Serienproduktion von Halbleiter-Sensoren kaum geeignet ist.

Bedingt durch die kurzen freien Weglängen der aufzustäubenden Teilchen und der hieraus resultierenden Stoßprozesse können auch bei der Verwendung von Schablonen keine exakten geometrischen Strukturen mit scharf abgegrenzten Randzonen hergestellt werden.

Ein weiterer gravierender Nachteil besteht darin, daß die Zahl der aufzustäubenden Substanzen begrenzt ist ; mehrfach dotierte Perowskite lassen sich auf diese Weise nicht herstellen.

Die bisher in der Literatur beschriebenen Perowskit-Halbleiter weisen, wie Untersuchungen gezeigt haben, über einen größeren Meßgaspartialdruckbereich von einigen Größenordnungen gesehen keine eindeutige

EP 0 365 567 B1

Kennlinie (Widerstandsänderung als Funktion der Meßgaskonzentration) auf. Meist durchläuft die Kennlinie ein Minimum, weshalb zwei verschiedene MeßgasKonzentrationsbereiche existieren, in denen der Halbleiter denselben elektrischen Widerstand aufweist.

Solche Halbleiter können nur in Meßgas-Konzentrationsbereichen eingesetzt werden, in denen die Kennlinie eindeutig verläuft.

Der Erfindung liegt die Aufgabe zugrunde, resistive Halbleiter für Gassensoren mit hoher Ansprechgeschwindigkeit herzustellen, die zur Messung des Partialdruckes von Sauerstoff und reduzierenden Gasen geeignet sind und deren Widerstandsänderung auf einem Volumeneffekt beruht.

Insbesondere soll die Schichtdicke dieser Halbleiter kleiner sein als die Schichtdicke bis jetzt durch Sinterung hergestellter resistiver Halbleiter.

Das Herstellungsverfahren soll in der Weise flexibel sein, daß Halbleiter stark verschiedener Zusammensetzung mit den selben Verfahrenshilfsmitteln hergestellt werden können. Weiterhin sollen die Halbleiter vorgegebene geometrischen Strukturen und scharf ausgeprägte Randzonen aufweisen.

Unter Verwendung von auf dieser Weise hergestellten Halbleitern sollen Gassensoren geschaffen werden, die für die Messung des Partialdrucks von Sauerstoff und reduzierenden Gasen in einem beliebig vorgegebenen Meßbereich zwischen $10^{-30}$ und ca. 1 bar bzw. in diesem gesamten Meßbereich geeignet sind.

Die Aufgabe wird erfindungsgemäß durch einen Halbleiter der allgemeinen Zusammensetzung $A'_xA_{1-x-z1}B'_yB_{1-y-z2}O_3$ gelöst, der dadurch gekennzeichnet ist, daß

a)     der Wert von x im Bereich von 0 bis 0,05, der Wert von y im Bereich von 0 bis 0,1 und die Absolutwerte von z1 und z2 im Bereich von 0 bis 0,002 liegen,

b)     der Halbleiter mit Hilfe der Dickfilmtechnik hergestellt ist, wobei die Schichtdicke weniger als 100 μm, vorzugsweise zwischen etwa 1 und 20 μm beträgt.

Hierbei ist

A'     mindestens ein Element der 1. Hauptgruppe, vorzugsweise Li, Na, K, Rb, und/oder mindestens ein Element der 3. Nebengruppe, vorzugsweise Y und/oder mindestens ein Element der Gruppe der Lanthaniden, vorzugsweise La ;

A     mindestens ein Element der 2. Hauptgruppe, vorzugsweise Ca, Sr, Ba ;

B'     mindestens ein Element der 2. Hauptgruppe, vorzugsweise Mg, und/oder mindestens ein Element der 3. Hauptgruppe, vorzugsweise Al, und/oder mindestens ein Element der Gruppe der Übergangsmetalle, vorzugsweise Mn, Cr, Fe, Co, Ni ;

B     ein Element der Gruppe Ti, Zr, Sn.

Das Verfahren zur Herstellung dieses Halbleiters ist dadurch gekennzeichnet, daß

a)     der Perowskit pulverisiert wird und mit einem organischen Pastengrundstoff zu einer Paste verarbeitet wird,

b)     die Paste mit Hilfe der Dickfilmtechnik auf ein Substrat aufgebracht wird, wobei eine Schichtdicke von weniger als 100 μm, vorzugsweise zwischen 1 und 20 μm, eingehalten wird,

c)     der durch Dickfilmtechnik aufgetragene Halbleiter einer Temperaturbehandlung unterzogen wird, derart, daß in einer ersten Aufheizphase die flüssigen Bestandteile des organischen Pastengrundstoffs verdampft werden, in einer zweiten Aufheizphase bei höheren Temperaturen die festen Bestandteile des organischen Pastengrundstoffs rückstandsfrei verbrannt werden und daß sich eine dritte Aufheizphase anschließt, deren Zeitdauer und Maximaltemperatur derart gewählt sind, daß ein Ablösen des Halbleitermaterials vom Substrat verhindert wird.

Die Dickfilmtechnik ist in besonderer Weise geeignet, Halbleiterschichtdicken unterhalb von 100 μm, insbesondere zwischen ca. 1 und 20 μm, zu realisieren, wodurch sich die Ansprechgeschwindigkeit solcher Gassensoren beträchtlich vergrößert.

Ein besonders geeignetes Dickfilmverfahren stellt die Siebdrucktechnik dar, die bisher hauptsächlich als hochwertiges Farbdruckverfahren für Papier, Stoff o.ä. Materialien Anwendung findet.

Weiterhin können als Dickfilmtechnik in vorteilhafter Weise das Tauchbadverfahren und das Aufsprühen von der aus pulverisiertem Mischoxid und organischem Pastengrundstoff bestehenden Paste unter Druck angewendet werden, wobei sich definierte geometrische Strukturen mit scharf abgegrenzten Randzonen unter Verwendung einer Schablone herstellen lassen.

Die Dickfilmtechnik stellt ein einfaches und wirkungsvolles Herstellungsverfahren dar, das insbesondere für eine Serienproduktion von Halbleiter-Sensoren geeignet ist, da die Betriebskosten dieses Verfahrens gegenüber der Sintertechnik und der Dünnfilmtechnik deutlich geringer sind und eine Nachbearbeitung entweder überhaupt nicht oder nur in geringem Maße notwendig ist.

Ein besonderer Vorteil besteht darin, daß Halbleitermaterialien der unterschiedlichsten Zusammensetzung mit einer beliebigen Anzahl von Komponenten mit denselben Verfahrenshilfsmitteln in Serienfertigung verarbeitet werden können. Dies ist insbesondere mit der Dünnfilmtechnik nicht möglich.

Durch die Dotierung von Perowskiten und durch gezielte Abweichungen von der Stöchiometrie bzw. durch die Verwendung von mindestens zwei verschiedenen Halbleitern können Gassensoren hergestellt werden, die in einem bestimmten vorgegebenen oder in einem viele Zehnerpotenzen umfassenden Meßbereich Verwendung finden können.

Die Erfindung wird anhand der folgenden Beispiele näher erläutert :

Beispiel 1

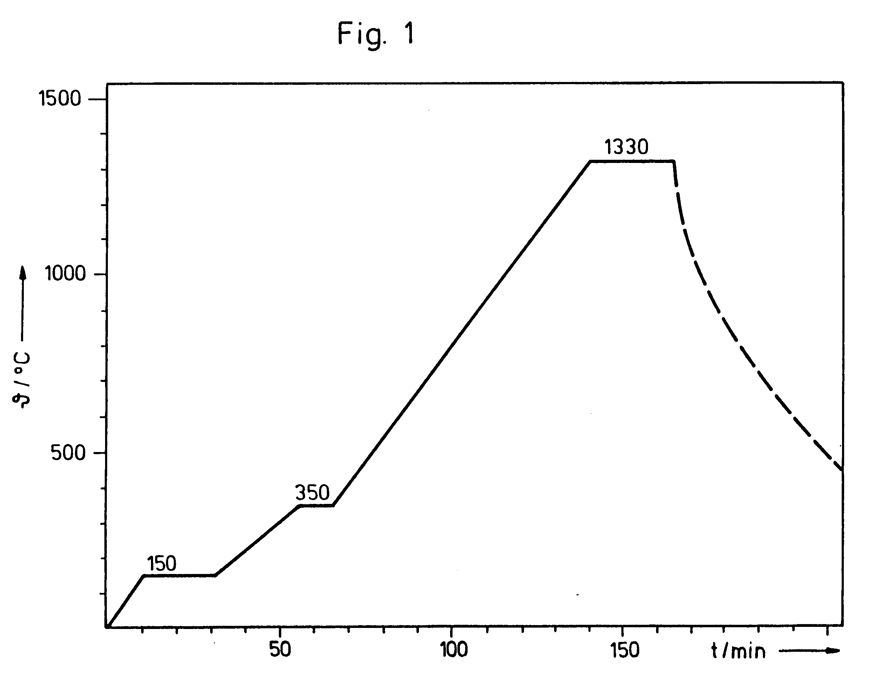

Herstellen der Paste aus 70% $SrTiO_3$ (HST-2/HPST-2 von Fuji) und 30% Pastengrundstoff (bestehend aus Ethylcellulose, Butylcarbitolacetat und $\alpha$-Terpineol). Auftragen der Schicht mittels Siebdruck, danach Durchfahren eines Temperaturprofils bis 1330°C entsprechend Fig. 1.

Beispiel 2

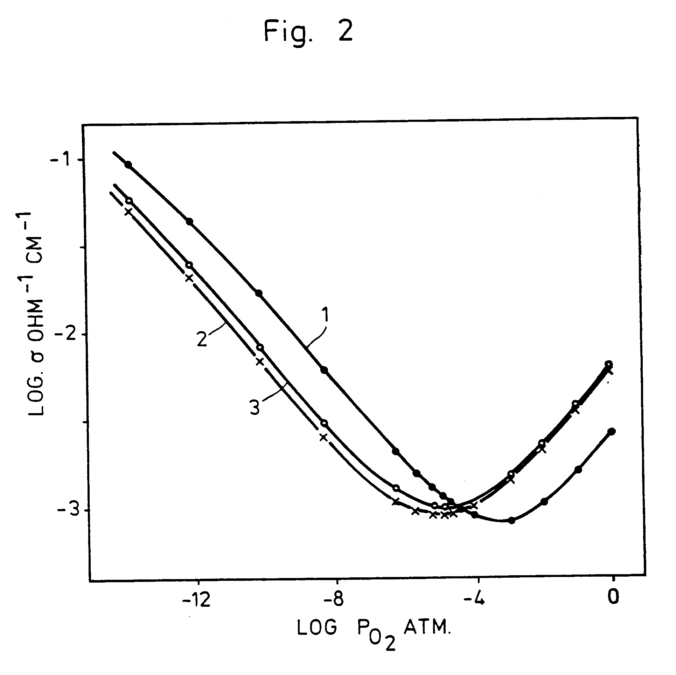

In der Figur 2 wird die Verschiebung des Kennlinienminimums und damit des Meßbereichs eines Halbleiter-Sensors durch gezielte Abweichungen von der Stöchiometrie dargestellt.

Aufgetragen sind die Kennlinien von Perowskit-Halbleitern mit einem Sr/Ti-Verhältnis von 1,0000 (Kurve 1), von 0,9990 (Kurve 2) und 0,9950 (Kurve 3) bei 1000°C.

Beispiel 3

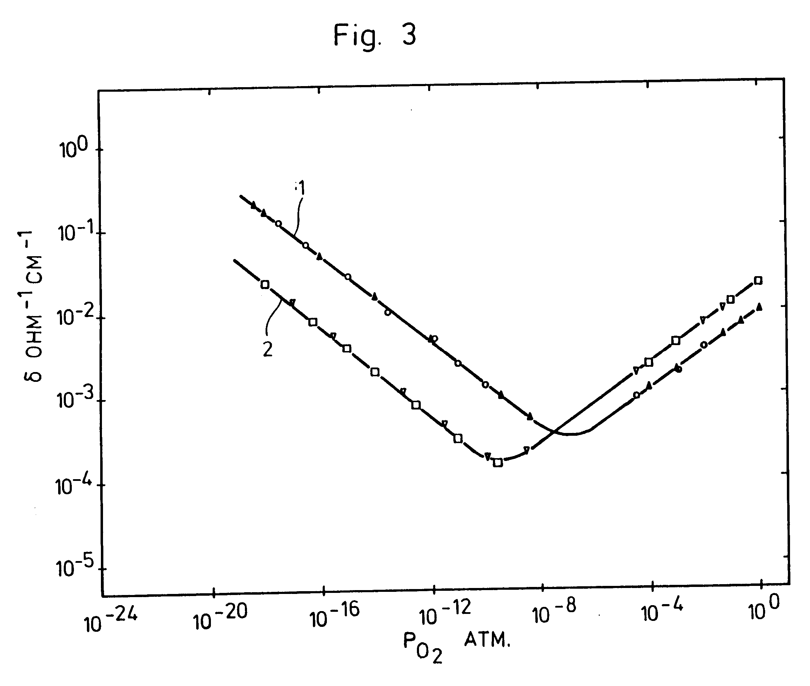

In der Fig. 3 wird die Verschiebung des Kennlinienminimums und damit des Meßbereichs eines Halbleiter-Sensors durch gezielte Dotierung mit Chrom bzw. Aluminium dargestellt. Die Versuchstemperatur beträgt 1000°C. Kurve 1 zeigt die Kennlinie von $Ca\,Cr_{0,0025}\,Ti_{0,9975}\,O_3$, die mit der Kennlinie von $Ca\,Al_{0,0025}\,Ti_{0,9975}\,O_3$ zusammenfällt, Kurve 2 zeigt die Kennlinie von $Ca\,Cr_{0,0055}\,Ti_{0,9945}\,O_3$, die mit der Kennlinie von $Ca\,Al_{0,0055}\,Ti_{0,0045}\,O_3$ zusammenfällt.

Beispiel 4

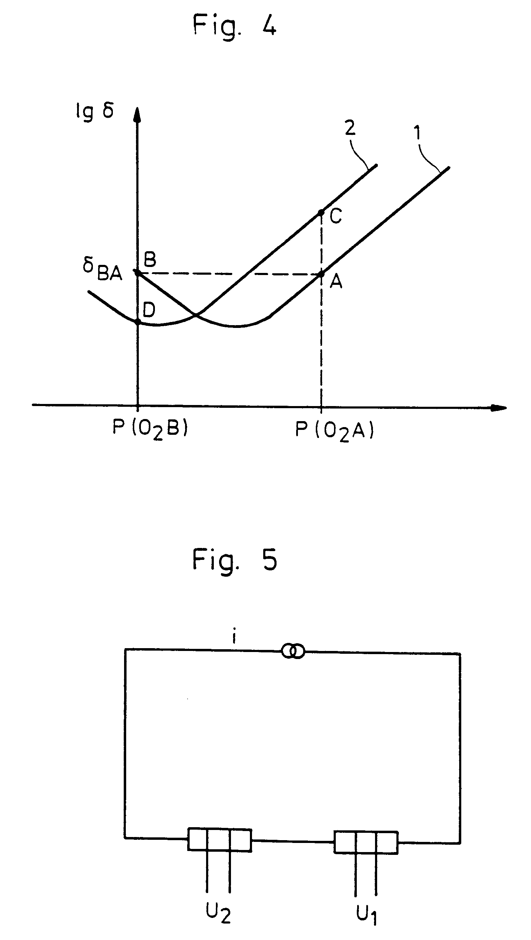

Gegeben sei ein perowskitischer Halbleiter mit einer im Meßbereich nicht eindeutigen Kennlinie für Sauerstoff entsprechend der Kennlinie 1 in Fig. 4. Ein anderer Halbleiter 2 weise eine Kennlinie 2 auf, die ein Minimum bei einer geringeren Sauerstoffkonzentration als Kennlinie 1 aufweist. Ein solcher Halbleiter kann beispielsweise durch Akzeptordotierung des Halbleiters 1, d.h. durch Ersatz eines Teils des Elements A durch ein einwertiges Element der Gruppe A' hergestellt werden.

Soll $p(O_2A)$ detektiert werden, so kann der von Kennlinie 1 gelieferte Wert $\sigma_{BA}$ sowohl von Punkt A als auch von Punkt B herrühren. Wird nun ein zweiter Halbleiter z.B. mit höherer Akzeptordotierung in Reihe geschaltet, so kann mit dessen Kennlinie 2 festgestellt werden, welcher der beiden Punkte A oder B den Leitfähigkeitswert $\sigma_{BA}$ liefert : Gibt der Halbleiter 2 eine höhere Spannung $U_2$ ab als der Halbleiter 1 ($U_2 > U_1$), so wird $p(O_2A)$ detektiert ; liefert 2 eine niedrigere Spannung ($U_2 < U_1$), so wird $p(O_2B)$ erkannt. Das zugehörige Prinzipschaltbild ist in Fig. 5 dargestellt.

Beispiel 5

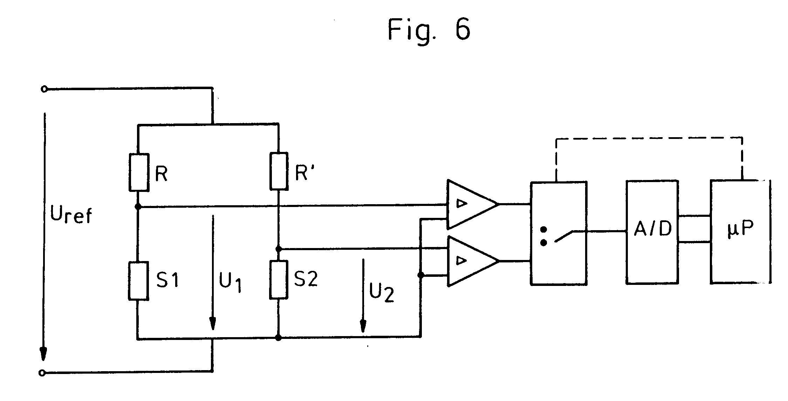

Ein weiteres Beispiel für die Messung einer Gaskomponente mit Hilfe mehrerer Halbleiter zeigt die in Fig. 6 skizzierte Anordnung.

Zur Messung reduzierender Gase (in diesem Fall Kohlenmonoxid) wird einer von zwei identischen Halbleitersensoren mit einer für die Totaloxidation katalytisch aktiven-Schicht überzogen. Als katalytisch aktive Schicht eignet sich eine dünne, poröse Platinschicht.

Die Anordnung besteht aus zwei Sensorwiderständen S1 und S2 und zwei bekannten, identischen Widerständen R und R'. Über die Vorgabe einer Referenzspannung $U_{ref}$ und den zu messenden Spannungen $U_1$ und $U_2$ läßt sich die Differenz der Sensorwiderstände nachfolgender Formel :

$$R = S1 - S2$$

$$= R\left(\frac{U_1}{U_{ref} - U_1} - \frac{U_2}{U_{ref} - U_2}\right)$$

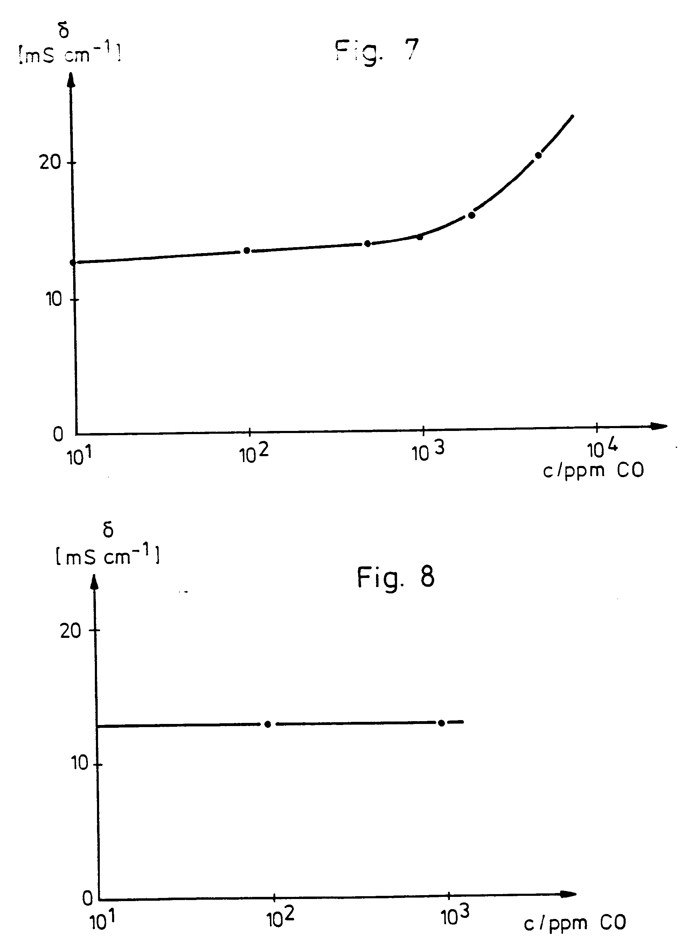

Fig. 7 zeigt die Abhängigkeit der Sensorleitfähigkeit von der Kohlenmonoxidkonzentration bei einer Temperatur von 1000°C und einem Sauerstoffgehalt von 5% in Stickstoff bei einem $SrTiO_3$-Halbleiter.

Fig. 8 zeigt, daß unter sonst gleichen Bedingungen ein mit einer katalytischen Deckschicht versehener Sensor eine vom Kohlenmonoxidgehalt unabhängige Leitfähigkeit besitzt.

Eine Anordnung entsprechend Fig. 6 ist insbesondere in der Lage, auch bei schwankendem Sauerstoffgehalt in der Gasphase selektiv die Konzentration der reduzierenden Gaskomponente zu bestimmen.

**Patentansprüche**

1. Halbleiter für einen resistiven Gassensor mit hoher Ansprechgeschwindigkeit zur Bestimmung des Partialdrucks von Sauerstoff und reduzierenden Gasen aus einem dotierten Perowskit der allgemeinen Zusammensetzung $A'_x A_{1-x-z1} B'_y B_{1-y-z2} O_3$, wobei A', A, B' und B Elemente und die Parameter z1 und z2 Stöchiometrieindices bedeuten, dadurch gekennzeichnet, daß

a) der Wert von x im Bereich von 0 bis 0,05, der Wert von y im Bereich von 0 bis 0,1 und die Absolutwerte von z1 und z2 im Bereich von 0 bis 0,002 liegen,

b) der Halbleiter mit Hilfe der Dickfilmtechnik hergestellt ist, wobei die Schichtdicke weniger als 100 µm, vorzugsweise zwischen etwa 1 und 20 µm beträgt.

2. Verfahren zur Herstellung eines Halbleiters nach Anspruch 1, dadurch gekennzeichnet, daß

a) der Perowskit pulverisiert wird und mit einem organischen Pastengrundstoff zu einer Paste verarbeitet wird,

b) die Paste mit Hilfe der Dickfilmtechnik auf ein Substrat aufgebracht wird, wobei eine Schichtdicke von weniger als 100 µm, vorzugsweise zwischen 1 und 20 µm, eingehalten wird,

c) der durch Dickfilmtechnik aufgetragene Halbleiter einer Temperaturbehandlung unterzogen wird, derart, daß in einer ersten Aufheizphase die flüssigen Bestandteile des organischen Pastengrundstoffs verdampft werden, in einer zweiten Aufheizphase bei höheren Temperaturen die festen Bestandteile des organischen Pastengrundstoffs rückstandsfrei verbrannt werden und daß sich eine dritte Aufheizphase anschließt, deren Zeitdauer und Maximaltemperatur derart gewählt sind, daß ein Ablösen des Halbleitermaterials vom Substrat verhindert wird.

3. Verfahren nach Anspruch 2, dadurch gekennzeichnet, daß als Dickfilmtechnik das Siebdruckverfahren angewendet wird.

4. Verfahren nach Anspruch 2, dadurch gekennzeichnet, daß als Dickfilmtechnik die Tauchbadbeschichtung angewendet wird, wobei das Substrat mit einer Schablone maskiert wird.

5. Verfahren nach Anspruch 2, dadurch gekennzeichnet, daß als Dickfilmtechnik ein Verfahren angewandt wird, bei dem das pastöse Halbleitermaterial unter Druck auf das mit einer Schablone maskierte Substrat aufgesprüht wird.

6. Verfahren nach Anspruch 2, dadurch gekennzeichnet, daß durch Änderung der Dotierung mindestens eines Elements der Gruppe A' und/oder B' oder durch Änderung der Stöchiometrieindices z1 und/oder z2 die Kennlinie des Halbleiters im Meßbereich eindeutig gestaltet wird.

7. Verfahren nach Anspruch 2, dadurch gekennzeichnet, daß zur Bestimmung des Partialdrucks von Sauerstoff und reduzierenden Gasen im Meßbereich zwischen $10^{-30}$ und 1 bar

a) mindestens zwei verschiedene Halbleiter verwendet werden und die elektrische Widerstände dieser Halbleiter zur Bestimmung des Meßgaspartialdruckes herangezogen werden,

b) die Halbleiter sukzessive durch wiederholte Anwendung der Dickfilmtechnik auf ein Substrat aufgebracht werden, wobei die Schichtdicken weniger als 100 µm, vorzugsweise zwischen 1 und 20 µm betragen.

## Claims

1. Semiconductor for a resistive gas sensor with a high response speed for determining the partial pressure of oxygen and reducing gases from a doped perowskite of the general formula $A'_xA_{1-x-z1}B'_yB'_{1-y-z2}O_3$, where $A'$, A, $B'$ and B are elements and the parameters z1 and z2 represent stoichiometric indices, characterised in that

a)     the value of x is in the range from 0 to 0.05, the value of y is in the range from 0 to 0.1 and the absolute values of z1 and z2 lie in the range from 0 to 0.002,

b)     the semiconductor is produced using the thick film technique, the thickness of the layer being less than 100 μm, preferably between about 1 and 20 μm.

2. Method of manufacturing a semiconductor according to claim 1, characterised in that

a)     the perowskite is pulverised and formed into a paste with an organic paste base,

b)     the paste is applied to a substrate using the thick film technique, resulting in a layer thickness of less than 100 μm, preferably between 1 and 20 μm,

c)     the semiconductor applied by the thick film technique is subjected to a temperature treatment in such a way that in a first heating phase the fluid components of the organic paste base material are evaporated, in a second heating phase at a higher temperature the solid components of the organic paste base material are burnt off without residue and that finally there is a third heating phase of which the duration and maximum temperature are chosen to be such that detachment of the semiconductor material from the substrate is prevented.

3. Method according to claim 2, characterised in that the screen printing process is employed as the thick film technique.

4. Method according to claim 2, characterised in that the immersion coating method is employed as the thick film technique, the substrate being masked with a stencil.

5. Method according to claim 2, characterised in that there is employed as the thick film technique a process in which the paste semiconductor material is sprayed under pressure onto the substrate which is masked with a stencil.

6. Method according to claim 2, characterised in that by altering the doping of at least one element of the group $A'$ and/or $B'$ or by altering the stoichiometric indices z1 and/or z2 the characteristic of the semiconductor in the measuring range is clearly defined.

7. Method according to claim 2, characterised in that for determining the partial pressure of oxygen and reducing gases in the range of measurement between $10^{-30}$ and 1 bar

a)     at least two different semiconductors are employed and the electrical resistances of these semiconductors are obtained to determine the partial pressure of the gas to be measured,

b)     the semiconductor is applied onto a substrate by repeated use of the thick film technique, the thicknesses of the layers amounting to less than 100 μm, preferably between 1 and 20 μm.

## Revendications

1. Semi-conducteur pour un capteur résistif de gaz à grande vitesse de réponse, destiné à déterminer la pression partielle de l'oxygène et de gaz réducteurs à partir d'une perovskite dopée ayant la composition générale $A'_xA_{1-x-z1}B'_yB_{1-y-z2}O_3$, où $A'$, A, $B'$ et B sont des éléments, et les paramètres z1 et z2 représentent les indices stoechiométriques, caractérisé en ce que :

a)     x est compris entre 0 et 0,05, y est compris entre 0 et 0,1, et les valeurs absolues de z1 et z2 sont comprises entre 0 et 0,002,

b)     le semi-conducteur est fabriqué par la technique des couches épaisses, l'épaisseur de couche étant inférieure à 100 μm et de préférence comprise entre 1 et 20 μm.

2. Procédé de fabrication d'un semi-conducteur selon la revendication 1, caractérisé en ce que

a)     on pulvérise la perovskite et on la façonne en une pâte à l'aide d'une matière de base organique pour pâte,

6

b)   on applique la pâte sur un substrat par la technique des couches épaisses, en respectant une épaisseur de couche inférieure à 100 μm, de préférence comprise entre 1 et 20 μm,

c)   le semi-conducteur appliqué par la technique des couches épaisses est soumis à un traitement thermique, de telle sorte que, dans une première phase d'échauffement, les constituants liquides de la matière de base organique pour pâte sont vaporisés, dans une deuxième phase d'échauffement à des températures plus élevées, les constituants solides de la matière de base organique pour pâte sont brûlés sans résidu, une troisième phase d'échauffement ayant lieu ensuite, dont on choisit la durée et la température maximale de façon à empêcher que le matériau semi-conducteur ne se détache du substrat.

3. Procédé selon la revendication 2, caractérisé en ce qu'on utilise en tant que technique des couches épaisses le procédé de sérigraphie.

4. Procédé selon la revendication 2, caractérisé en ce qu'on utilise comme technique des couches épaisses un revêtement par immersion, le substrat étant masqué par un pochoir.

5. Procédé selon la revendication 2, caractérisé en ce qu'on utilise comme technique des couches épaisses un procédé dans lequel le matériau semi-conducteur pâteux est pulvérisé sous pression sur le substrat masqué par un pochoir.

6. Procédé selon la revendication 2, caractérisé en ce que, par une modification du dopage d'au moins un élément du groupe A' et/ou B', ou par modification de l'indice stoechiométrique z1 et/ou z2, on donne à la courbe caractéristique du semi-conducteur dans l'intervalle de mesure un caractère monotone.

7. Procédé selon la revendication 2, caractérisé en ce que, pour déterminer la pression partielle de l'oxygène et de gaz réducteur dans l'intervalle de mesure compris entre $10^{-30}$ et 1 bar,

a)   on utilise au moins deux semi-conducteurs différents, et les résistances électriques de ces semi-conducteurs sont utilisées pour déterminer la pression partielle du gaz de mesure,

b)   les semi-conducteurs sont successivement appliqués sur un substrat par utilisation répétée de la technique des couches épaisses, les couches épaisses ayant une épaisseur inférieure à 100 μm et de préférence comprise entre 1 et 20 μm.

## Fig. 1

EP 0 365 567 B1

Fig. 2

Fig. 3

EP 0 365 567 B1

# Fig. 4

# Fig. 5

Fig. 6

Fig. 7

Fig. 8